# EUROPEAN PATENT APPLICATION

(11) **EP 2 656 861 A1**
(43) Date of publication of application: **30.10.2013**
(21) Application number: 13165087.1
(22) Date of filing: 24.04.2013
(51) Int. Cl.: A61L 9/12

(54) **Device for vaporizing substances**

(30) Priority: 27.04.2012 ES 201230460 U
(71) Applicant: Zyxtudio diseño e innovación SL, 46003 Valencia (ES)
(72) Inventor: BLASCO FEO, VICENTE, 46003 Valencia (ES)
(74) Representative: Soler Lerma, Santiago

(57) **Abstract**

Device for vaporizing substances of those contained in a vessel that is closed on the top by a breathable membrane fitted horizontally, characterised in that it comprises at least one body open on its sides, with a preferably tubular shape (1), which can house the vessel containing the substance, means (5) for preventing the rotation of the vessel inside the body open on its sides, and means (4) for deforming the vessel, thereby bringing into action the tearing elements.

## Description

The present invention relates to a device for facilitating the vaporization of substances to the atmosphere, specifically substances contained in a vessel closed on its top part by a breathable membrane.

It belongs to the field of air fresheners and disinsection.

### BACKGROUND

The patent literature includes many systems for facilitating the evaporation of substances to the atmosphere, among which are those comprising a vessel with the substance to evaporate and a breathable membrane that gradually allows the exit of the substance to distribute.

The most commonly used types of membrane are those of polyolefin, and more recently silica.

Patent EP1988934 discloses a vessel in which the membrane is in a top horizontal position, closing the vessel.

The substance to vaporize does not have to be in contact with the membrane, but if the membrane is impregnated with this substance, such as when shaking the device, the vaporization intensity increases as the membrane is impregnated.

In these devices the vaporization is constant, as the vaporization surface is constant, but if a greater vaporization is desired at a given time it can be obtained by impregnating the membrane, such as by shaking the vessel.

This type of vaporizers comprise an outer vessel closed on the top by the breathable membrane and an inner vessel, normally placed upside-down and normally closed on its bottom by a film that can be ripped easily.

The outer vessel is provided on its base with a number of sharp elements placed opposite the aforementioned easily torn film, so that pressing the base of the outer vessel will make these sharp elements tear the film, allowing the substance to exit the inner vessel into the outer vessel, and its vaporization to the atmosphere through the membrane.

The patent literature also includes document WO2010/120961 relating to a method for delivering a volatile substance to the atmosphere which comprises a vessel containing the substance to vaporize, closed by a membrane, provided with an inner film that must be torn to release the substance and allow it to reach the atmosphere.

In this patent the membrane is in a lateral position, with an inner reservoir provided to bring the membrane close to the liquid to vaporize.

Complementing the above-cited patent is WO2010/120960, which discloses a casing in which to introduce the vessel, force the tearing of the inner film and keep the vessel in a vertical position, favoring the contact of the substance with the membrane.

Similar solutions can be found in patents WO99/03514 or WO98/16262.

### DESCRIPTION OF THE INVENTION

The object of the present invention is to provide this vessel with a casing that makes it easier to tear the inner film and to place the vessel in different locations of interest, or to shake it when greater vaporization is required.

For this purpose, the device comprises:
- A casing that allows housing the vessel with the substance.
- Means to prevent the vessel from rotating inside the casing.
- Means for deforming the vessel, actuating the sharp elements.

The casing has a substantially tubular shape. The vessel is introduced on the side of the casing, with its path limited by longitudinal guides that meet the upper edges of the vessel, keeping it from rotating inside the casing.

The casing has a top opening or chimney to facilitate the vaporization of the substance in the vessel.

In its ends, or at least in one of its ends, the casing has a narrowing near its top in the form of a neck so that, when the vessel is introduced and as it cannot rotate, it will be deformed making the sharp elements tear the film that closes the inner vessel.

This narrowing also prevents the inner vessel from falling out when the device is shaken to obtain a momentary greater vaporization.

To facilitate the use of this casing in different situations, it is provided with various complements, some of which are described below by way of example.

For its use in a household, on a piece of furniture or a shelf, it is provided with flange-like protrusions defining some support legs.

For its use in cars, it is provided with means for attachment to the profiles of laminar parts, such as ventilation grilles, said means comprising a clip and an articulated joint, such as a ball joint, that allows a sideways oscillation of the casing.

To help the casing return to its initial position after said oscillation, the rotation shaft is placed eccentrically in height but longitudinally centered in the casing.

For use in closed premises, particularly bathrooms, an element for attaching it to a wall is foreseen of the type comprising a surface with an adhesive and a shaft, parallel to the wall, that allows an anterior-posterior oscillation of the casing.

### BRIEF DESCRIPTION OF THE DRAWINGS

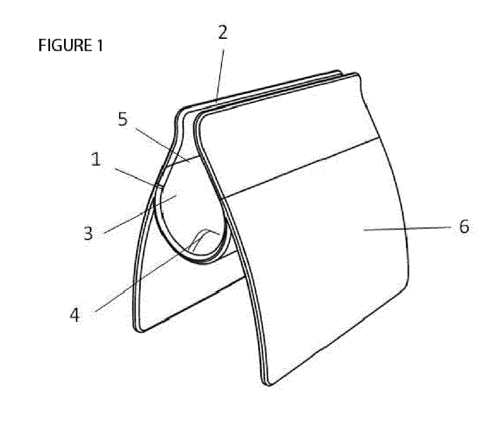

FIGURE 1 shows a general view of the casing of the device in an embodiment intended for use in households, to be placed on a surface, with a substantially tubular body (1) having a grooved opening on its top part (2) and side openings (3) allowing the vessel (not shown) to be introduced, the protrusion that narrows the side inlets (4) and deforms the vessel when it is introduced, the stops that hold the vessel on its top part (5) and the bottom extensions that facilitate the stability of the assembly when it is resting on a surface (6).

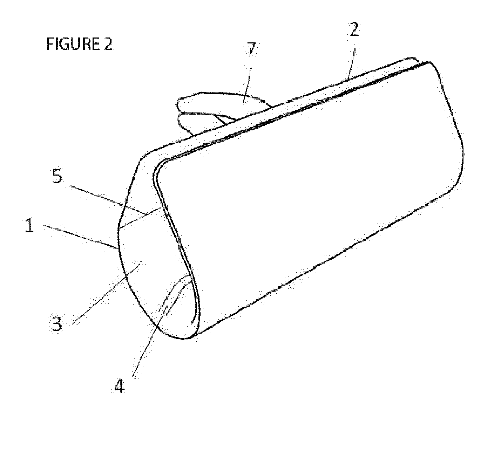

FIGURE 2 shows a general view of the casing of the device in an embodiment intended for use in cars, showing the substantially tubular body (1) having a grooved opening on its top part (2) and side openings (3) allowing the vessel (not shown) to be introduced, the protrusion that narrows the side inlets (4) and deforms the vessel when it is introduced, the stops that hold the vessel on its top part (5) and the addition of a clip (7) for attachment to laminar bodies such as the air conditioning grilles of cars.

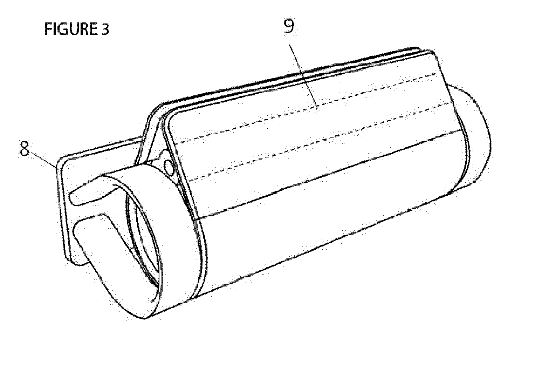

FIGURE 3 shows a general view of the casing of the device in an embodiment intended for use by attachment to a vertical surface, such as in a bathroom, showing the same common elements as in the previous figures as well as an attachment and swinging element that comprises an area for attachment to a smooth vertical surface (8) with a shaft that longitudinally crosses the vessel internally (9).

### DESCRIPTION OF AN EMBODIMENT OF THE INVENTION

An embodiment of is described herein by way of example that is not meant to limit the invention in any way.

The device comprises
a) a main body consisting of:
   a. A tubular casing (1) open on its sides (3) that can house inside it a vessel containing the substance to vaporize.
   b. Inside the casing, crossing it in a longitudinal sense, some guides (5) that act as a stop and receive the upper edges of the vessel.
   c. Some individual narrowings (4) at the side openings of the casing.
   d. The casing is provided on its top part with a longitudinal opening (2).
b) some attachment means (6) consisting of some legs defining a bottom flange for the casing.

To use it a cartridge containing the substance to evaporate is inserted in the casing.

These cartridges comprise an outer reservoir and an inner reservoir.

The outer reservoir is closed by a microporous membrane, while the inner reservoir is closed by a film that can be easily torn.

The vessel has elements allowing to tear the aforementioned easily-torn film, these elements being located near said easily-torn film.

The narrowings of the side openings of the casing force the vessel to deform when it is inserted in the casing, such that the tearing elements will tear the easily-torn film and allow the substance contained in the inner reservoir to exit to the outer reservoir, from where it will exit gradually to the atmosphere through the microporous membrane.

The shape of the device allows it to be placed both horizontally and vertically.

During its use evaporation will be constant, but if greater vaporization is desired at a given time the casing can be shaken so that the membrane is impregnated, leading to greater vaporization.

## Claims

1. DEVICE FOR VAPORIZING SUBSTANCES of the type contained in a vessel closed on its top by a breathable membrane placed horizontally, **characterised in that** it comprises:
• A body open on its sides, with a preferably tubular shape (1) that can house the vessel containing the substance.
• Means (5) for preventing the rotation of the vessel inside the body open on its sides.
• Means (4) for deforming the vessel, bringing into action the tearing elements.

2. DEVICE FOR VAPORIZING SUBSTANCES according to claim 1, **characterized in that** the casing has an opening (2) on its top part.

3. DEVICE FOR VAPORIZING SUBSTANCES according to claim 1, **characterized in that** the casing has means for physically supporting it on a flat surface (6).

4. DEVICE FOR VAPORIZING SUBSTANCES according to claim 1, **characterized in that** the casing has means for attaching it to the profile of a laminar surface (7).

5. DEVICE FOR VAPORIZING SUBSTANCES according to claim 4, **characterized in that** the means for attaching the casing to the profile of a laminar surface comprise a clip.

6. DEVICE FOR VAPORIZING SUBSTANCES according to claim 4, **characterized in that** the means for attaching the casing to the profile of a laminar surface are joined to the casing by a hinged element that conforms the transverse shaft about which an oscillatory movement can take place.

7. DEVICE FOR VAPORIZING SUBSTANCES according to claim 6, **characterized in that** the hinged element that allows the oscillation referred to in claim 6 is a ball joint.

8. DEVICE FOR VAPORIZING SUBSTANCES according to claim 4, **characterized in that** the shaft referred to in claim 6 is present in the top part of the casing.

9. DEVICE FOR VAPORIZING SUBSTANCES according to claim 1, **characterized in that** the casing has means (8) for attaching it to a vertical surface.

10. DEVICE FOR VAPORIZING SUBSTANCES according to claim 9, **characterized in that** the means for attaching the device to a vertical surface comprise a horizontal shaft parallel to the vertical surface, which crosses the casing in a longitudinal sense allowing a transverse oscillation.

11. DEVICE FOR VAPORIZING SUBSTANCES according to claim 1, **characterized in that** at least one of the open sides has a narrowing.

12. DEVICE FOR VAPORIZING SUBSTANCES according to claim 1, **characterized in that** the narrowest part of said narrowing has a height that is less than that of the vessel, so that in order to pass through said point the vessel must be deformed.
